# FASCICULE DE BREVET EUROPEEN

(11) **EP 1 355 981 B1**
(45) Date de publication et mention de la délivrance du brevet: **01.04.2009**
(21) Numéro de dépôt: 02700371.4
(22) Date de dépôt: 22.01.2002
(51) Int. Cl.: C08L 5/00, C08L 5/04, C08L 5/08, C08L 5/12, A61L 27/26, A61L 27/20

(54) **COMPOSITION GELIFIABLE, BIOCOMPATIBLE ET IMPLANTABLE**
GELIERBARE, BIOKOMPATIBLE UND IMPLANTIERBARE ZUSAMMENSETZUNG
IMPLANTABLE AND BIOCOMPATIBLE GEL COMPOSITION

(30) Priorité: 22.01.2001 FR 0100832
(43) Date de publication de la demande: 29.10.2003
(73) Titulaire: Laboratoire TBF, 69500 Bron (FR)
(72) Inventeur: BARDONNET, Rapha¬l, 77240 Seine-Port (FR); LAGANIER, Laurent, F-69390 Vernaison (FR)
(74) Mandataire: Guerre, Dominique
(86) Numéro de dépôt international: PCT/FR2002/000262
(87) Numéro de publication internationale: WO 2002/057355

(56) Documents cités:
- WO-A-91/01720
- WO-A-98/40111
- WO-A-99/15211
- US-A- 6 060 053
- DATABASE CHEMABS [en ligne] chemical abstracts service, columbus, ohio, us; "Gel hardmness of gel made from alginates" retrieved from STN Database accession no. 68:56734 CA XP002178518 & SATO TERUHIKO: HOKUSUISHI GEPPO, vol. 24, no. 9, 1967, pages 350-356, jp
- PATENT ABSTRACTS OF JAPAN vol. 012, no. 191 (C-501), 3 juin 1988 (1988-06-03) & JP 62 296853 A (KIBUN KK;OTHERS: 01), 24 décembre 1987 (1987-12-24)

## Description

La présente invention concerne la formulation et la mise en forme de compositions gélifiables biocompatibles permettant de réaliser des implants.

Dans le domaine de la cicatrisation des tissus comme les cartilages ou les tissus osseux, il est souvent nécessaire de recourir à des greffes de chondrocytes.

Ces greffes de chondrocytes utilisent à ce jour des suspensions cellulaires parfois difficiles à utiliser, il en résulte un besoin de produit ou de moyen permettant de réaliser ces greffes de chondrocytes par la mise en oeuvre de matériaux solides, présentant des caractéristiques mécaniques permettant une manipulation aisée.

On connaît les matériaux biodégradables que sont par exemple les polymères naturels comme les celluloses, les méthytcelluloses, les gélatines, les caséines, les chitosanes, les polylysines et les autres polysaccharides.

On connaît également les propriétés par exemple de gélification de certains de ces biomatériaux comme les polysaccharides permettant la réalisation de produits solides, et leur biocompatibilité suffisante pour maintenir la viabilité de cellules. Il est ainsi connu de maintenir en vie des cellules dans des milieux contenant des polysaccharides naturels et l'inclusion de matériel vivant ou de principes actifs pharmaceutiques dans des gels constitués de polysaccharides est donc possible.

Les polysaccharides naturels macromoléculaires sont composés de molécules de saccharides non hydrolysées liées entre elles pour former des chaînes longues.

Les macromolécules peuvent être composées d'environ 3 000 molécules de saccharides et de préférence de 300 à 1 200 molécules.

Différents moyens et procédés ont été mis en oeuvre pour obtenir des gels d'alginates utilisables comme matériau implantable susceptible de contenir ou non des extraits tissulaires vivants et/ou des principes actifs pharmaceutiques.

On connaît par exemple de W09825053, un gel d'alginate composé d'une suspension de particules d'hydrogel ayant un diamètre compris entre 30 et 500 µm.

On connaît de GB2201966 un matériau composite constitué de deux composés gélifiables dont une partie de l'un des deux composés gélifiables est éliminée permettant ainsi de former un gel composite poreux présentant des cavités contenant par exemple des cellules.

On connaît de la publication Hokusuishi Geppo (1967), 24, 350-6, l'influence de solutions de sels de cations métalliques sur la dureté de gels constitués d'agar et d'alginate de sodium.

Plus récemment, pour des applications dans le domaine alimentaire l'utilisation de composés dont le processus de gélification est thermo réversible en mélange avec de l'alginate de sodium a été révélée dans JP62296853 pour produire des gels susceptibles de résister à des températures élevées, tout en étant peu déformables.

Les polysaccharides naturels macromoléculaires sont composés de molécules de saccharides non hydrolysées liées entre elles pour former des chaînes longues.

Les macromolécules peuvent être composées d'environ 3 000 molécules de saccharides et de préférence de 300 à 1 200 molécules.

Parmi les solutions précédemment mises en oeuvre, aucune n'a à ce jour permis de préparer des implants présentant des propriétés mécaniques compatibles avec leur utilisation lors de gestes chirurgicaux et de manipulation aisée.

Il est par exemple connu d'utiliser des gels d'alginates. Les alginates sont des dérivés de l'algine, qui est un polysaccharide complexe présent dans les parois cellulaires d'une algue brune appartenant au groupe des phaetophyceae. L'alginate est extrait des algues sous forme d'une solution sel de sodium.

Les alginates présentent notamment la propriété de former des gels par réaction avec des cations divalents. Ces gels sont en général constitués d'environ 97,5 à 99% d'eau et de 0,3 à 2,5% de polymères.

L'inclusion de matériel vivant ou de principes actifs pharmaceutiques est possible, notamment, il est connu d'utiliser les gels d'alginate pour maintenir en suspension des cellules. Ces dernières peuvent par exemple être dispersées dans une solution d'alginate qui sera polymérisée au contact d'une solution de sel de calcium ou de barium.

Cependant ce mode de polymérisation ne permet pas de former un bloc de gel pouvant être mis en forme, ni le moulage. On obtient des billes indépendantes d'un gel qui présente néanmoins des caractéristiques mécaniques intéressantes, car il n'est pas friable, il est manipulable et même découpable.

Parmi les autres polysaccharides utilisables on connaît l'agarose, qui est dérivé de l'agar qui est extrait de différentes sortes d'algues rouges appartenant au groupe des rhodophyceae, c'est un galactane hydrocolloïde linéaire.

L'agar et les agaroses ont la propriété de former des gels par refroidissement ; ces gels présentent des propriétés physiques variables en fonction de l'origine des agars ou des agaroses. Ces polysaccharides sont donc facilement mis en forme grâce à leurs propriétés de gélification en fonction de la température, par exemple par moulage à chaud. Ils peuvent également en travaillant à partir de solution être moulés à une température supérieure à la température de gélification puis après refroidissement être démoulables.

Ces agars ou agaroses ont également la particularité de présenter des températures de gélification inférieures à 40°C, compatibles avec la vie cellulaire et inférieures aux températures de dégradation de nombreux principes actifs pharmaceutiques. Cependant les caractéristiques mécaniques de ces gels sont insuffisantes car ils sont friables et cassants et ne résistent pas aux contraintes imposées lors de la réalisation de gestes chirurgicaux.

La présente invention, permet la réalisation d'implants présentant des propriétés mécaniques compatibles avec leur utilisation notamment lors de gestes chirurgicaux et permettant la greffe de chondrocytes.

La présente invention concerne une composition aqueuse de polysaccharides, gélifiable et biocompatible **caractérisée en ce qu**'elle comprend un mélange d'au moins deux polysaccharides dont l'un est gélifiable par voie chimique et l'autre par voie thermique.

Un polysaccharide gélifiable est ainsi un polysaccharide présentant la propriété de former un gel, par réticulation comme les alginates, les agaroses, les chitosanes.

La réticulation est effectuée, en fonction de la nature des polysaccharides mis en oeuvre, par abaissement de la température ou par addition d'une solution saline, par exemple contenant des ions calcium ou barium ou modification du pH.

On entend ici par gélifiable par voie thermique l'aptitude pour une composition à former un gel par abaissement de la température.

On entend ici par gélifiable par voie chimique, l'aptitude pour une composition à former un gel par réaction par exemple avec des cations métalliques qui provoquent une réticulation et donc une formation de gel ou par modification du pH de la solution de polysaccharide.

Les polysaccharides gélifiables par voie chimique selon l'invention sont choisis parmi les alginates et les chitosanes.

Les chitosanes sont constitués de résidus N-acétylglucosamine unis par des liaisons béta-1,4, dérivés par désacétylation de la chitine qui forme de longues chaînes. La chitine est le constituant principal de l'exosquelette des crustacés.

Le collagène est une scléroprotéine complexe, insoluble dans l'eau et les solutions salines, se transformant en gélatine sous l'action de l'eau bouillante, formée de macromolécules de tropocollagène. Le collagène est le composant essentiel des fibrilles et fibres du tissu conjonctif.

Les polysaccharides gélifiables par voie thermique selon l'invention sont l'agar ou l'agarose.

Les deux polysaccharides préférés selon l'invention sont l'agarose et l'alginate.

Les compositions aqueuses de polysaccharides selon l'invention contiennent entre 0.5 et 10 % de polysaccharide gélifiable chimiquement et entre 0,5 et 10 % de polysaccharide gélifiable thermiquement.

La présente invention concerne une composition aqueuse de polysaccharides, gélifiable et biocompatible dont l'un est gélifiable chimiquement et l'autre thermiquement ; ce mélange permettant par formation du gel par réticulation par abaissement de la température de créer une structure moulable et solide permettant ensuite par réticulation chimique du deuxième polysaccharide d'améliorer les propriétés mécaniques.

La présente invention concerne une composition aqueuse de polysaccharides, gélifiable et biocompatible dont l'un est gélifiable chimiquement et l'autre thermiquement, **caractérisée en ce qu**'elle comprend des extraits tissulaires biologiques vivants ou des cellules autologues, allogéniques ou xénogéniques

Les cellules susceptibles d'être mise en oeuvre dans une composition selon l'invention appartiennent au groupe des chondrocytes et autres cellules constitutives du cartilage, au groupe des ostéoblastes ou autres cellules constitutives des os, au groupe des cellules musculaires ou fibroblastes.

Ces cellules seront de préférence choisies parmi les cellules capables de stimuler la régénération des tissus osseux ou cartilagineux.

Les cellules capables de stimuler la régénération des tissus cartilagineux sont par exemple des chondrocytes isolés et multipliés par culture cellulaire à partie de biopsie de cartilage.

Les compositions gélifiables biocompatibles préparées selon l'invention peuvent contenir également de l'os spongieux déminéralisé.

Les compositions gélifiables biocompatibles préparées selon l'invention peuvent contenir également des agents susceptibles de traiter ou de prévenir des pathologies ou des complications.

Ces agents sont par exemple des principes actifs pharmaceutiques comme par exemple des agents antiviraux ou des agents antibactériens.

Ces agents peuvent être présents dans les compositions gélifiables biocompatibles selon l'invention seuls ou en mélanges.

Les compositions selon l'invention sont préparées par le procédé comprenant les étapes suivantes :
- solubilisation dans l'eau du polymère gélifiable par voie chimique,
- addition du polysaccharide gélifiable par voie thermique et mise en solution par chauffage
- stérilisation et refroidissement.

On obtient un gel intermédiaire, partiellement réticulé qui éventuellement, est remis en solution par chauffage avant addition des extraits fissulaires biologiques, d'os spongieux déminéralisé ou des principes actifs pharmaceutiques.
- refroidissement du mélange obtenu pour gélification par voie thermique et moulage, on obtient donc une forme intermédiaire qui ne présente pas les propriétés mécaniques requises pour l'utilisation.
- démoulage puis gélification par réticulation chimique par mise en contact avec une solution saline.

Ce procédé permet en outre d'obtenir si nécessaire des gels Intermédiaires stérilisables, prêts à l'emploi pour l'incorporation de principes actifs et/ou de suspensions cellulaires.

Dans une variante de préparation, la gélification par vole chimique peut être réalisée in situ au moment de l'implantation ou après l'implantation par addition d'une solution saline.

Les solutions salines selon l'invention sont de préférence des solutions de sels de calcium ou de barlum.

La présente invention concerne également le procédé de fabrication d'un obturateur fémoral, **caractérisé en ce qu**'il comporte au moins les étapes suivantes :
- solubilisation dans l'eau du polymère gélifiable par voie chimique,
- addition du polysaccharide géliflable par voie thermique et mise en solution par chauffage
- refroidissement du mélange obtenu pour gélification par vole thermique et moulage,
- démoulage puis gélification par réticulation chimique par mise en contact avec une solution saline.

### Exemples d'application :

### 1. Implant contenant des chondrocytes destiné à la réparation du cartilage articulaire

### Fabrication d'un Implant contenant des chondrocytes

L'implant est formé de chondrocytes cultivés, d'un gel composite et éventuellement, d'os spongieux déminéralisé humain.

### Préparation des chondrocytes

Les chondrocytes proviennent d'une biopsie de cartilage réalisée sur un patient atteint de lésions chondrales ou ostéo-chondrales. Après Isolement grâce à un traitement enzymatique approprié, les chondrocytes sont multipliés en culture cellulaire jusqu'à obtention d'un nombre suffisant. L'ensemble des étapes d'obtention des chondrocytes est réalisé en milieu aseptique.

Ces cellules sont alors récupérées et mises en suspension dans du milieu de culture avant la fabrication de l'implant.

### Préparation de l'os spongieux déminéralisé :

Le tissu osseux humain provient d'une banque de tissus. Il subit une étape de dévitalisation et de viro-inactivation par action chimique et physique. Le tissu osseux est ajusté à la forme désirée puis déminéralisé en milieu acide à 4°C. L'état stérile est obtenu par rayonnement gamma.

### Préparation du gel composite :

1 Dissoudre de l'alginate dans une solution de Hank's à température ambiante à une concentration généralement comprise entre 1 et 10%.
2 Suspendre de l'agarose de type "low gelling" dans le mélange précédent à une concentration généralement comprise entre 1 et 10%
3 Après homogénéisation, chauffer immédiatement l'ensemble à 90°C afin de dissoudre l'agarose en suspension.
4 Stériliser par autoclavage à 121°C pendant 20 minutes la solution d'alginate et d'agarose.
5 Le gel composite se forme après refroidissement à température de gélification de l'agarose.

### Préparation de l'implant

L'ensemble des opérations se déroule dans des conditions aseptiques.
1 liquéfier le gel composite par chauffage à 90°C puis le maintenir à 37°C pour son utilisation.
2 Ajouter la suspension cellulaire.
3 Après homogénéisation, couler le mélange dans un moule.
4 Pour les implants avec phase osseuse, l'os spongieux déminéralisé est alors rajouté selon les besoins.
5 Porter ensuite le moule à 4°C pendant 10 minutes pour permettre à l'implant de gélifier.
6 Démouler l'implant puis le plonger 10 minutes dans une solution de CaCl₂ 25 mM pour transformer l'alginate de sodium en alginate de calcium.
7 Incuber l'implant à 37°C, 5% de CO₂ dans du milieu DMEM ajusté en CaCl₂ à 3 mM jusqu'à utilisation.

### Variante de préparation

Lorsque l'implant ne contient pas d'os déminéralisé, il est possible de transformer l'alginate de sodium en alginate de calcium seulement in situ au moment de l'implantation. L'implant est alors utilisé comme une pâte qui est mise en forme dans la lésion à traiter. Par aspersion d'une solution contenant 25 mM de chlorure de calcium, l'implant se solidifie in situ en adoptant la forme de la lésion

### II. Obturateur fémoral destiné à faciliter la mise sous pression du ciment chirurgical et à limiter sa progression dans le canal diaphysaire du fût fémoral lors de la pose de prothèses de hanche

Les obturateurs habituellement utilisés sont soit en matériaux synthétiques biodégradables (polymère d'acide lactique ou glycolique), soit à base de gélatine d'origine animale (porcine en général). Dans le premier cas, la lyse du polymère entraîne des phénomènes inflammatoires qui ne sont pas souhaitables. Dans le deuxième cas, l'utilisation de produit d'origine animale représente un risque potentiel de transmission de maladies infectieuses (prions).

### Fabrication d'un obturateur fémoral

1 Dissoudre de l'alginate à une concentration généralement comprise entre 1 et 10% dans de l'eau pour préparation injectable.
2 Mettre en suspension de l'agarose "Hight gelling" à une concentration généralement comprise entre 1 et 10%.
3 Dans un mélangeur, porter immédiatement le mélange à une température supérieure à la température de fusion de l'agarose tout en homogénéisant.
4 Couler le gel dans les moules pour obturateur préchauffés à 50°C
5 Laisser refroidir les moules 30 minutes à 4°C puis démouler.
6 Plonger les obturateurs 12 heures dans une solution de CaCl₂ 100 mM
7 Rincer deux fois une heure les obturateurs dans du soluté physiologique de qualité injectable.

## Revendications

1. Composition aqueuse de polysaccharides gélifiable et biocompatible constituée d'un mélange d'au moins deux polysaccharides dont l'un est gélifiable par voie chimique et l'autre par voie thermique, **caractérisée en ce qu'**elle contient des extraits tissulaires biologiques.

2. Composition selon la revendication 1, **caractérisée en ce que** les extraits tissulaires biologiques sont des cellules autologues, allogéniques ou xenogéniques.

3. Composition selon la revendication 2, **caractérisée en ce que** les cellules autologues, allogéniques ou xenogéniques appartiennent au groupe des chondrocytes et autres cellules constitutives du cartilage, au groupe des ostéoblastes ou autres cellules constitutives des os, au groupe des cellules musculaires ou des fibroblastes.

4. Composition selon, la revendication 1, **caractérisée en ce qu'**elle contient un principe actif pharmaceutique seul ou en mélange.

5. Composition selon la revendication 1, **caractérisée en ce qu'**elle contient de l'os spongieux déminéralisé.

6. Composition selon l'une quelconque des revendications précédentes **caractérisée en ce que** le polysaccharide gélifiable par voie chimique est choisi parmi les alginates ou les chitosanes.

7. Composition selon l'une quelconque des revendications précédentes **caractérisée en ce que** le polysaccharide gélifiable par voie thermique est choisi parmi l'agarose et l'agar.

8. Composition selon l'une quelconque des revendications précédentes **caractérisée en ce que** les polysaccharides sont présents respectivement dans le gel à une concentration comprise entre 0,5 et 10 %.

9. Implant **caractérisé en ce qu'**il est susceptible d'être obtenu par gélification d'une composition selon l'une quelconque des revendications 1 à 8.

10. Utilisation d'une composition aqueuse de polysaccharides gélifiable et biocompatible constituée d'un mélange d'au moins deux polysaccharides dont l'un est gélifiable par voie chimique et l'autre par voie thermique pour la préparation d'implant.

11. Utilisation d'une composition aqueuse de polysaccharides gélifiable et biocompatible constituée d'un mélange d'au moins deux polysaccharides dont l'un est gélifiable par voie chimique et l'autre par voie thermique pour la réalisation d'obturateur fémoral.

12. Utilisation selon l'une quelconque des revendications 10 ou 11, **caractérisée en ce que** le polysaccharide gélifiable par voie chimique est choisi parmi les alginates ou les chitosanes.

13. Utilisation selon l'une quelconque des revendications 10 ou 11, **caractérisée en ce que** le polysaccharide gélifiable par voie thermique est Choisi parmi l'agarose et l'agar.

14. Procédé de préparation d'une composition aqueuse de polysaccharides, gélifiable et biocompatible, **caractérisé en ce qu'**il comporte les étapes suivantes :
- solubilisation dans l'eau du polysacharide gélifiable par voie chimique,
- addition du polysaccharide gélifiable par voie thermique et mise en solution par chauffage,
- stérilisation et refroidissement,
- obtention d'un gel intermédiaire partiellement réticulé,
- solubilisation du gel intermédiaire par chauffage,
- addition des principes actifs pharmaceutiques et/ou des extraits tissulaires biologiques et/ou d'os spongieux déminéralisé,
- refroidissement du mélange obtenu pour gélification par voie thermique et moulage,
- démoulage puis gélification par réticulation chimique par mise en contact avec une solution saline.

15. Procédé de préparation d'un obturateur fémoral, **caractérisé en ce qu'**il comporte au moins les étapes suivantes :
- solubilisation dans l'eau du polysaccharide gélifiable par voie chimique,
- addition du polysaccharide gélifiable par voie thermique et mise en solution par chauffage
- refroidissement du mélange obtenu pour gélification par voie thermique et moulage,
- démoulage puis gélification par réticulation chimique par mise en contact avec une solution saline.

## Claims

1. Biocompatible and gellable aqueous composition of polysaccharides consisting of a mixture of at least two polysaccharides, one of which is chemically gellable and the other of which is thermally gellable, **characterized in that** it contains biological tissue extracts.

2. Composition according to Claim 1, **characterized in that** the biological tissue extracts are autologous, allogenic or xenogenic cells.

3. Composition according to Claim 2, **characterized in that** the autologous, allogenic or xenogenic cells belong to the group of chondrocytes and other cells constituting cartilage, to the group of osteoblasts or other cells constituting bone, to the group of muscle cells or fibroblasts.

4. Composition according to Claim 1, **characterized in that** it contains a pharmaceutical active principle alone or as a mixture.

5. Composition according to Claim 1, **characterized in that** it contains demineralized spongy bone.

6. Composition according to any one of the preceding claims, **characterized in that** the chemically gellable polysaccharide is chosen from alginates or chitosans.

7. Composition according to any one of the preceding claims, **characterized in that** the thermally gellable polysaccharide is chosen from agarose and agar.

8. Composition according to any one of the preceding claims, **characterized in that** the polysaccharides are respectively present in the gel at a concentration between 0.5 and 10%.

9. Implant, **characterized in that** it can be obtained by gelling of a composition according to any one of Claims 1 to 8.

10. Use of a biocompatible and gellable aqueous composition of polysaccharides consisting of a mixture of at least two polysaccharides, one of which is chemically gellable and the other of which is thermally gellable, for preparing an implant.

11. Use of a biocompatible and gellable aqueous composition of polysaccharides consisting of a mixture of at least two polysaccharides, one of which is chemically gellable and the other of which is thermally gellable, for producing a femoral obturator.

12. Use according to either one of Claims 10 and 11, **characterized in that** the chemically gellable polysaccharide is chosen from alginates or chitosans.

13. Use according to either one of Claims 10 and 11, **characterized in that** the thermally gellable polysaccharide is chosen from agarose and agar.

14. Method for preparing a biocompatible and gellable aqueous composition of polysaccharides, **characterized in that** it comprises the following steps:
- solubilization of the chemically gellable polysaccharide in water,
- addition of the thermally gellable polysaccharide and dissolving by heating,
- sterilization and cooling,
- production of a partially crosslinked intermediate gel,
- solubilization of the intermediate gel by heating,
- addition of the pharmaceutical active principles and/or of the biological tissue extracts and/or of demineralized spongy bone,
- cooling of the mixture obtained for thermal gelling and molding,
- removal from the mold and then gelling by chemical crosslinking, by bringing into contact with a saline solution.

15. Method for preparing a femoral obturator, **characterized in that** it comprises at least the following steps:
- solubilization of the chemically gellable polysaccharide in water,
- addition of the thermally gellable polysaccharide and dissolving by heating,
- cooling of the mixture obtained for thermal gelling and molding,
- removal from the mold and then gelling by chemical crosslinking, by bringing into contact with a saline solution.

## Patentansprüche

1. Gelierbare und biokompatible wässrige Zusammensetzung von Polysacchariden, bestehend aus einem Gemisch von mindestens zwei Polysacchariden, von denen das eine auf chemischem Weg und das andere auf thermischem Weg gelierbar ist, **dadurch gekennzeichnet, dass** sie biologische Gewebeextrakte enthält.

2. Zusammensetzung nach Anspruch 1, **dadurch gekennzeichnet, dass** die biologischen Gewebeextrakte autologe, allogene oder xerogene Zellen sind.

3. Zusammensetzung nach Anspruch 2, **dadurch gekennzeichnet, dass** die autologen, allogenen oder xerogenen Zellen zur Gruppe der Chondrozyten und anderer knorpelbildender Zellen, zur Gruppe der Osteoblasten oder anderer knochenbildender Zellen, zur Gruppe der Muskelzellen oder der Fibroblasten gehören.

4. Zusammensetzung nach Anspruch 1, **dadurch gekennzeichnet, dass** sie einen pharmazeutischen Wirkstoff, allein oder im Gemisch, enthält.

5. Zusammensetzung nach Anspruch 1, **dadurch gekennzeichnet, dass** sie demineralisierten Schwammknochen enthält.

6. Zusammensetzung nach einem der vorhergehenden Ansprüche, **dadurch gekennzeichnet, dass** das auf chemischem Weg gelierbare Polysaccharid aus Alginaten oder Chitosanen ausgewählt ist.

7. Zusammensetzung nach einem der vorhergehenden Ansprüche, **dadurch gekennzeichnet, dass** das auf thermischem Weg gelierbare Polysaccharid aus Agarose und Agar ausgewählt ist.

8. Zusammensetzung nach einem der vorhergehenden Ansprüche, **dadurch gekennzeichnet, dass** die Polysaccharide in dem Gel jeweils in einer Konzentration zwischen 0,5 und 10% vorliegen.

9. Implantat, **dadurch gekennzeichnet, dass** es durch Gelieren einer Zusammensetzung nach einem der Ansprüche 1 bis 8 erhalten werden kann.

10. Verwendung einer gelierbaren und biokompatiblen wässrigen Zusammensetzung von Polysacchariden, die aus einem Gemisch von mindestens zwei Polysacchariden besteht, von denen das eine auf chemischem Weg und das andere auf thermischem Weg gelierbar ist, zur Herstellung eines Implantats.

11. Verwendung einer gelierbaren und biokompatiblen wässrigen Zusammensetzung von Polysacchariden, die aus einem Gemisch von mindestens zwei Polysacchariden besteht, von denen das eine auf chemischem Weg und das andere auf thermischem Weg gelierbar ist, zur Herstellung eines Femur-Obturatormuskels.

12. Verwendung nach einem der Ansprüche 10 oder 11, **dadurch gekennzeichnet, dass** das auf chemischem Weg gelierbare Polysaccharid aus Alginaten oder Chitosanen ausgewählt ist.

13. Verwendung nach einem der Ansprüche 10 oder 11, **dadurch gekennzeichnet, dass** das auf thermischem Weg gelierbare Polysaccharid aus Agarose und Agar ausgewählt ist.

14. Verfahren zur Herstellung einer gelierbaren und biokompatiblen wässrigen Zusammensetzung von Polysacchariden, **dadurch gekennzeichnet, dass** es folgende Schritte umfasst:
- Solubilisieren des auf chemischem Weg gelierbaren Polysaccharids in Wasser,
- Zugeben des auf thermischem Weg gelierbaren Polysaccharids und Lösen mittels Erhitzen,
- Sterilisieren und Abkühlen,
- Gewinnen eines teilweise vernetzten Gel-Zwischenprodukts,
- Solubilisieren des Gel-Zwischenprodukts mittels Erhitzen,
- Zugeben der pharmazeutischen Wirkstoffe und/oder der biologischen Gewebeextrakte und/oder von demineralisiertem Schwammknochen,
- Abkühlen des erhaltenen Gemischs für die Gelierung auf thermischem Weg und Formgießen,
- aus der Form Nehmen und anschließendes Gelieren mittels chemischer Vernetzung durch Zusammenbringen mit einer Salzlösung.

15. Verfahren zur Herstellung eines Femur-Obturatormuskels, **dadurch gekennzeichnet, dass** es mindestens die folgenden Schritte umfasst:
- Solubilisieren des auf chemischem Weg gelierbaren Polysaccharids in Wasser,
- Zugeben des auf thermischem Weg gelierbaren Polysaccharids und Lösen mittels Erhitzen,
- Abkühlen des erhaltenen Gemischs für die Gelierung auf thermischem Weg und Formgießen,
- aus der Form Nehmen und anschließendes Gelieren mittels chemischer Vernetzung durch Zusammenbringen mit einer Salzlösung.
